# EUROPÄISCHE PATENTANMELDUNG

(11) **EP 2 572 752 A1**
(43) Veröffentlichungstag der Anmeldung: **27.03.2013**
(21) Anmeldenummer: 12180767.1
(22) Anmeldetag: 17.08.2012
(51) Int. Cl.: A61N 1/08

(54) **Elektrodenkathetereinrichtung**

(30) Priorität: 21.09.2011 US 201161537073 P
(71) Anmelder: BIOTRONIK SE & Co. KG, 12359 Berlin (DE)
(72) Erfinder: Diebold, Michael, 12169 Berlin (DE); Weiss, Ingo, 12435 Berlin (DE); Hoffmeister, Sabine, 59075 Hamm (DE); Fandrey, Stephan, 8910 Affoltern am Albis (CH)
(74) Vertreter: Lindner-Vogt, Karin L.

(57) **Zusammenfassung**

Elektrodenkathetereinrichtung, die eine Leiterstruktur mit mindestens einer Elektrode enthält, mit Strommessmitteln zur Messung eines durch ein äußeres elektromagnetisches Feld in der Leiterstruktur induzierten Stromes, wobei die Strommessmittel zur lokalen Strommessung, insbesondere von Stromstärken und -phasen in der oder einer Elektrode oder einem vorbestimmten Bereich der Leiterstruktur ausgebildet und an einen mit dem äußeren Feld nicht wechselwirkenden Signalübertragungskanal angeschlossen sind.

## Beschreibung

Die Erfindung betrifft eine Elektrodenkathetereinrichtung, die eine Leiterstruktur mit mindestens einer Elektrode. Sie betrifft des Weiteren eine elektromedizinische Vorrichtung, die eine solche Elektrodenkathetereinrichtung umfasst.

Derartige Elektrodenkathetereinrichtungen sind insbesondere Stimmulationselektrodenleitungen (mitunter verkürzt auch als "Elektroden" bezeichnet) von Herzschrittmachern oder Schockelektrodenleitungen von implantierbaren Defibrillatoren, aber auch Katheter, die eine langgestreckte leitfähige Struktur enthalten.

Medizinische Implantate wie die erwähnten Schrittmacher und Defibrillatoren besitzen häufig eine elektrische Verbindung zum Körperinneren des Patienten. Eine solche Verbindung dient zur Messung von elektrischen Signalen, zur Stimulation, zur Verödung und/oder zur Defibrillation von Körperzellen. Diese Verbindung ist of als längliche Elektrode ausgeführt. Gegenwärtig werden elektrische Signale zwischen dem Implantat und den Elektrodenkontakten (Spitze, Ringe, HV-Schockwendeln, Sensoren o. Ä.) mit elektrisch gut leitenden Materialien übertragen.

Wird ein System aus Implantat und Elektrode starken Störfeldern (EMI, MRI) ausgesetzt, so kann es zu unerwünschtem Fehlverhalten kommen, speziell einer Erwärmung von Teilen des Systems oder elektrischen Fehlfunktionen (z. B. Resets). Die Erwärmung kann zu Schädigungen von Körpergewebe oder von Organen führen, wenn die erwärmten Teile direkten Kontakt zum Gewebe haben. Dies ist insbesondere bei der Elektrodenspitze der Fall.

Die Ursache für das unerwünschte Fehlverhalten ist die Wechselwirkung des Feldes mit der länglichen Leitungsstruktur der Elektrode: Die Elektrode wirkt als eine Antenne und empfängt Energie aus den umgebenden Feldern. Diese Energie auf den therapeutisch genutzten Leitungen kann die Antenne distal über die Elektrodenkontakte (Spitze, Ring...) an das Gewebe oder proximal an das Implantat abgeben.

Die gleichen Probleme treten auch bei anderen länglichen leitfähigen Strukturen auf, deren proximales Ende nicht zwingend mit einem Implantat verbunden ist (z.B. bei Kathetern, temporäre Elektroden etc.).

Die kritischste Interferenz ist der Resonanzeffekt, der unter anderem durch Abschirmung oder den Einsatz von Mantelsperren minimiert und durch optische oder induktive Entkopplung verhindert werden kann. Der erwähnte Einsatz von Mantelsperren wird etwa beschrieben in "Reduction of Resonant RF Heating in Intravascular Catheters Using Coaxial Chokes" Magn Reson Med. 43(4);615-9, April 2010, die Möglichkeit der optischen Entkopplung wird bspw. beschreiben in US 6, 925,322 oder in "Magnetic Resonance Safety Testing of a Newly-Developed Fiber-Optics Cardiac Pacing Lead", J Magn Reson Imaging. 16(1); 97-103 Juli 2002, und die Möglichkeit einer induktiven Entkopplung wird bspw. beschreiben in "Multifunctional Interventional Devices for MRI: A Combined Electrophysiology/MRI Catheter" Magn Reson Med. 47(3);594-600, März 2002, oder "Feasibility of Real-Time Magnetic Resonance Imaging for Catheter Guidance in Electrophysiology Studies" Circ 2008:118-223-227.

Die Abschirmung oder der Einsatz von Mantelwellensperren können eine effektive Reduzierung der HF-induzierten Effekte erreichen. Hierbei muss jedoch jede mögliche Position und Lage im MRT und im Patienten berücksichtigt werden. Damit kann eine unbeabsichtigte HF-Einkopplung nie ausgeschlossen werden.

Bei der Anmelderin werden Katheterprototypen entwickelt, bei denen eine metallische Leiterstruktur durch Kohlenstofffasern ersetzt ist oder bei der nichtmagnetische Materialien zum Einsatz kommen; vgl. zu diesen Optionen auch "Feasibility of Real-Time MRI With a Novel Carbon Catheter for Interventional Electrophysiology", 2009/3 Circ 2009:2:258-267 bzw. "Interactive Real-Time Mapping and Catheter Ablation of the Cavotricuspid Isthmus Guided by Magnetic Resonance Imaging in a Porcine Model", 2009/11 Eur. Heart Journal.

Des Weiteren wird hingewiesen auf Veröffentlichungen, die sich mit einer in eine Katheterspitze integrierten MR-Sonde befassen, nämlich auf S. Fandrey, S. Weiss, J. Müller "Development of an Active Intravascular MR-Device with an Optical Transmission System, IEEE Transactions on Medical Imaging 2008;27;1723 ― 1727 und "Aktive Magnetresonanz-Sonde in Mikrosystemtechnik auf Basis einer optischen Signalübertragung für die minimal-invasive Chirurgie", Verlag Dr. Hut, München 2010. Hierin wird die Integration eines elektrisch optischen Wandlers inklusive einer optischen Energieversorgung in einen 6F-Katheter beschrieben. Diese Sonde erlaubt eine generelle MR-Signalübertragung für Lokalisations- und Bildgebungszwecke in einem Gefäßsystem.

Die möglichen Interferenzen zwischen MR-Gerät und kritisch langen Leitungsstrukturen (wie u. a. EP Kathetern) und bekannte Lösungsansätze, die sich mit der Sicherheit beschäftigen, wurden bereits wissenschaftlich diskutiert. Im Übrigen wird auch in der Veröffentlichung M.G. Zanchi, R. Venook, J. Pauly, G Scott "An Optically Coupled System for Quantitative Monitoring of MRI-Induced RF Currents Into Long Conductors, IEEE Transaktion On Medical Imaging, Vol. 29, No. 1, January 2010 eine Lösung zur Messung von MRI-induzierten HF-Strömen in langen Leitern mittels eines Messsystems mit optischer Kopplung wissenschaftlich erörtert.

Der Erfindung liegt die Aufgabe zugrunde, eine verbesserte Elektrodenkathetereinrichtung zu schaffen, welche so ausgerüstet ist, dass mit ihr Störungen, die beim Einsatz in starken äußeren elektromagnetischen Feldern entstehen können, präziser erfasst werden können, um somit exakt bestimmte Gegenmaßnahmen treffen zu können.

Zur Anwendung der vorgeschlagenen Elektrodenkathetereinrichtung bzw. elektromedizinischen Vorrichtung ist eine einmalige Kalibrierungsmessung für ein spezifisches Katheter oder Elektrodensystem notwendig. Bei dieser Kalibrierungsmessung wird der Zusammenhang zwischen dem HF-Strom und der Gewebeerhitzung mit Hilfe des Stromsensors und einem Temperatursensor aufgenommen. So kann später im Realbetrieb eine Abschätzung getroffen werden welche Ströme noch im zulässigen Bereich sind und welche Ströme eine schädliche HF-Erhitzung verursachen.

Durch MRT verursachte HF induzierte Ströme können zu für den Patienten gefährlichen Gewebeerhitzungen führen. Der Stromsensor detektiert Ströme, die aus der Tipelektrode und/oder den Ringelektroden austreten. Mit dem detektierten Strom kann der Gewebeerwärmung entgegengewirkt werden. Dies kann entweder durch das Abschalten oder Anpassen der HF-Leistung des MRT oder durch eine zusätzliche Kühlung der Katheterspitze geschehen.

Diese Aufgabe wird durch eine Elektrodenkathetereinrichtung mit den Merkmalen des Anspruchs 1 gelöst. Zweckmäßige Fortbildungen des Erfindungsgedankens sind Gegenstand der abhängigen Ansprüche. Des Weiteren wird eine elektromedizinische Vorrichtung mit den Merkmalen des Anspruchs 16 vorgeschlagen.

Die erfindungsgemäße Lösung verbessert die Sicherheit bzw. ermöglicht erst den Einsatz eines Katheters mit Elektroden oder Herzschrittmacher/ICD-Elektroden im MRT. Der Sensor ermöglicht dabei eine Gefahrensituation schnell zu erkennen und ermöglicht dabei unmittelbare Gegenmaßnahmen betreffend der HF-induzierten Gewebeerwärmung.

Generell hat die MRT als Bildgebungsverfahren einige Vorteile gegenüber dem Röntgen, insbesondere bei minimal invasiven Eingriffen oder bei der Bildgebung von Organen und Weichteilen. Mit einem MR-sicheren Ablationskatheter könnte man z. B. eine Echtzeitbildgebung von Läsionen kurz nach oder während der Ablation ausnutzen. Auch alte Läsionen lassen sich mit dem Bildgebungsverfahren darstellen. Zudem vereinfacht das MR-Bildgebungsverfahren die Navigation. Damit ist insgesamt eine höhere Erfolgsrate bei Ablationsprozeduren zu erwarten. Zudem gibt es bei dem MR-Bildgebungsverfahren keine ionisierende Strahlung welches hier als zusätzlicher Vorteil zum Röntgen genannt werden kann.

Mit der Erfindung wird einerseits der Einsatz von Strommessmitteln in der Elektrodenkathetereinrichtung vorgeschlagen, welche zur lokalen Strommessung, insbesondere von Stromstärke und -phase in der oder einer Elektrode oder einem anderen vorbestimmten Bereich der Leiterstruktur ausgebildet sind. Sie schließt weiter den Gedanken ein, dass diese Strommessmittel an einen mit dem äußeren Feld nicht wechselwirkenden Signalübertragungskanal angeschlossen sind.

Hiermit können insbesondere der sichere Betrieb des Katheters garantiert und Gefahrensituationen erkannt werden. Im Falle der Integration in eine Herzschrittmacher/ICD-Elektrode kann der Stromsensor die HF-induzierten Ströme erkennen und eine Warnung durch das Implantat über einen Kommunikationskanal senden. Neben den erwähnten Herzschrittmacher- und ICD-Elektrodenleitungen kann die Erfindung vorteilhaft auch bei Katheteranordnungen zur Nieren- oder zur Tumorablation sowie bei Anordnungen zur Neurostimulation eingesetzt werden. Auch rein sensorisch genutzte Elektrodenleitungen oder sowohl zur Übertragung von Sensorsignalen als auch zur Übertragung von Therapieströmen genutzte Leitungen sind (über ihre weit verbreitete Nutzung bei Herzschrittmacheranordnungen hinaus) ein sinnvolles Einsatzfeld der Erfindung.

In einer Ausführung der Erfindung ist den Strommessmitteln ein elektrisch/mechanischer Wandler zur Signalwandlung in ein mechanisches Signal zugeordnet und der Signalübertragungskanal zur mechanischen Signalübertragung ausgebildet. In einer hierzu alternativen Ausführung ist den Strommessmitteln ein elektrisch/optischer Wandler zur Signalwandlung in ein optisches Signal zugeordnet und der Signalübertragungskanal zur optischen Signalübertragung ausgebildet. Eine weitere Ausführung sieht vor, dass den Strommessmitteln ein elektrisch/thermischer Wandler zur Signalwandlung in ein thermisches Signal zugeordnet und der Signalübertragungskanal zur thermischen Signalübertragung ausgebildet ist.

Gemäß einem anderen Konzept zur weitgehenden Unterdrückung von Störungen im Übertragungskanal ist den Strommessmitteln ein elektrisch/elektrischer Wandler bzw. Modulator zur Wandlung des Ausgangssignals der Strommessmittel in bzw. zur Aufmodulation auf elektromagnetische Wellen in einem Frequenzbereich zugeordnet ist, der mit einem Frequenzspektrum des äußeren Feldes nicht wesentlich interferiert, und der Signalübertragungskanal ist zur elektromagnetischen Signalübertragung ausgebildet. Hierbei kann insbesondere der Signalübertragungskanal einen drahtlosen Übertragungsabschnitt aufweisen. Dies verhindert zuverlässig eine induktive Einkopplung von Störungen, muss aber mit Maßnahmen zur Verhinderung einer Interferenz der Signalübertragungs-Wellen mit Störwellen aus dem äußeren Feld einhergehen.

Besonders effizient gelingt dies, wenn dem elektrisch/elektrischen Wandler bzw. Modulator Kodierungsmittel zur Kodierung des Ausgangssignals der Strommessmittel zur Verringerung von Störeinflüssen aus dem äußeren elektromagnetischen Feld zugeordnet sind. Die Kodierung von Nutzsignalen hat sich nicht zuletzt in der Mobilfunktechnik als höchst wirkungsvolle Maßnahme zur Sicherung einer weitgehend störungsfreien und zuverlässigen Signalübertragung in hochgradig störungsbehafteten Umgebungen erwiesen, und dem Fachmann sind geeignete Kodierungsverfahren als solche bekannt.

Unabhängig von vorgenannten Maßnahmen, aber auch in Kombination hiermit, kann vorgesehen sein, dass der Signalübertragungskanal einen elektrischen Leiter aufweist, welcher in Abstimmung auf die geometrische Ausführung der Leiterstruktur aus derartigem Material gebildet und/oder derart geformt und/oder derart verlegt ist, dass nur eine geringe Kopplung mit dem äußeren elektromagnetischen Feld und der Leiterstruktur erfolgt.

Gemäß einer weiteren Ausführung der Erfindung ist den Strommessmitteln unmittelbar ein elektrischer Leiter als elektrischer Signalübertragungskanal oder Teil eines solchen zugeordnet, wobei die Strommessmittel und der zugeordnete Leiter insbesondere einstückig ausgebildet sind.

In einer weiteren Ausführung der Erfindung sind die Strommessmittel als passive oder energie-autark arbeitende Messmittel ausgebildet. Sie können bspw. unter Nutzung der Körperwärme eines Lebewesens betrieben werden, in das die Elektrodenkathetereinrichtung eingeführt ist. Auch Bewegungen des Lebewesens können zur Energieversorgung der Strommessmittel ausgenutzt werden. Auch die Nutzung von Energie aus dem externen Störfeld zum Betrieb der Strömmesseinrichtung kommt in Betracht.

In einer hierzu alternativen Ausführung sind die Strommessmittel als aktive Messmittel ausgebildet und an einen Energieübertragungskanal angeschlossen. In analoger Weise wie beim Signalübertragungskanal kann auch hier die Einbindung eines drahtlosen Übertragungsabschnitts vorgesehen sein. Im Übrigen kann der Energieübertragungskanal natürlich einen elektrischen Leiter, insbesondere Elektroden- oder Ionenleiter, aufweisen. Andererseits kann vorgesehen sein, dass der Energieübertragungskanal Mittel zur mechanischen, insbesondere hydraulischen oder pneumatischen, Energieübertragung oder Mittel zur optischen Energieübertragung, insbesondere eine Lichtleitfaser, aufweist.

Im Hinblick auf die vorstehend erwähnte aktive Ausführung der Strommessmittel kann vorgesehen sein, dass das Mess- bzw. Therapiegerät der vorgeschlagenen elektromedizinischen Vorrichtung eine an den Energieübertragungskanal der Elektrodenkathetereinrichtung anschließbare Energiequelle zur Energieversorgung der Strommessmittel aufweist.

In einer bevorzugten Ausführung der Vorrichtung weist das Mess- bzw. Therapiegerät eine eingangsseitig mit den Auswertungsmitteln verbundene Betriebssteuereinrichtung zur Steuerung eines Mess- und/oder Therapievorganges in Abhängigkeit vom Auswertungsergebnis des Ausgangssignals der Strommessmittel auf. Grundsätzlich lässt sich die mit der Erfindung verfolgte Zielstellung aber auch über eine gegenüber dem eigentlichen Mess- bzw. Therapiegerät externe Einflussnahme auf den Mess- bzw. Therapievorgang oder dessen Parameter erreichen, etwa durch geeignete Gestaltung einer zentralen, nachträglichen Auswertung oder durch Einflussnahme auf den Patienten außerhalb des eigentlichen Mess-oder Therapiekanals.

Aspekte von Ausführungen der Erfindung ergeben sich im Übrigen aus der nachfolgenden Auflistung.
- Der Stromsensor ist ein Wandler, der eine zum Strom proportionale (oder allgemein eine Funktion davon darstellende Größe) an eine Auswerteeinheit weiterleitet.
- Der Stromsensor ist zur Funktionsleitung in Reihe geschaltet und erfasst den Strom unmittelbar, ist z. B. als Widerstand oder allgemein Impedanz (d. h. auch als Kondensator oder Spule bzw. Übertrager) ausgebildet und erzeugt an seinen Enden einen Spannungsabfall proportional zum Strom. Das übertragene Messsignal ist also eine Spannung. Es ist auch der Einsatz eines nichtlinearen Widerstrandes möglich, der die Signalamplitude z. B. logarithmisch kodiert.
- Der Stromsensor wandelt den durch ihn fließenden Strom in ein akustisches/mechanisches (Piezo/Quarz), optisches (LED), pneumatisches/hydraulisches oder Temperatur-Signal.
- Der Stromsensor wandelt den durch ihn fließenden Strom in eine drahtlos (insbesondere auch über den Körper) übertragbare Welle, z. B. eine elektromagnetische Welle (in einem Frequenzbereich, der mit den Störfeldern nicht interferiert), oder akustische/Welle (in einem Frequenzbereich, der mit den Gradienten nicht interferiert).
- Der Stromsensor wandelt den Strom mittelbar, indem er das Magnetfeld bzw. den durch den Strom erzeugten magnetischen Fluss erfasst. Vorteil: Der Funktionsleiter muss nicht zertrennt werden, Zuverlässigkeitsprobleme mit Kontaktierungstechnik werden vermieden.
- Der Stromsensor besteht aus einem primären Wandler (z.B. Strom in Spannung) und einem sekundären Wandler, der z. B. diese Spannung in eine andere für die Übertragung zur Auswerteeinheit besser geeignete Größe verwandelt z. B. einen akustisches/mechanisches (Piezo/Quarz), optisches (LED), pneumatisches/hydraulisches oder Temperatur-Signal.
- Die Energie wird elektrisch (Elektronenleiter, Ionenleiter = Elektrolytschlauch), mechanisch/akustisch (Piezo), optisch (Laser / Licht), hydraulisch/pneumatisch zugeführt.
- Der Magnetfeldsensor ist eine Spule (i. allg. ein zweiter Leiter, der induktiv zum Funktionsleiter koppelt).
- Der Magnetfeldsensor ist ein Hall-Sensor, GMR-Sensor.
- Zur verbesserten Kopplung des Sensors an die Funktionsleiter sind diese oder zumindest einer davon am Ort der Strommessung geeignet geformt; z. B. auch wenn der Funktionsleiter sonst linear verläuft, ist er hier gewendelt, und die Sensorspule bildet die Sekundärwicklung dieses Überträgers. Verläuft der Funktionsleiter hingegen linear, ist der Sensor z. B. eine Toroidspule (Rogowski-Spule).
- Zur verbesserten Kopplung des Sensors an den die Funktionsleiter ist/sind diese/r oder zumindest einer davon am Ort der Strommessung mit geeigneten Materialeigenschaften versehen; z. B. sind die Sensorleiter und/oder Funktionsleiter hochleitend beschichtet, damit der entstehende Überträger weniger Verluste hat.

- Die Zuleitung vom Sensor zur Auswerteeinheit ist zwecks Verminderung der Kopplung an den Funktionsleiter oder externer Störungen als optischer Leiter, akustischer Leiter, hydraulisch/pneumatischer Leiter ausgebildet.
- Die Zuleitung vom Sensor zur Auswerteeinheit ist zwecks Verminderung der Kopplung an den Funktionsleiter oder der externen Störungen als elektrischer Leiter ausgebildet wobei die Verminderung der Kopplung durch geeignete Formung bzw. Führung der Leiter erfolgt (so dass sie minimal induktiv koppeln, bevorzugt möglichst geradlinig gestreckt, z. B. in einer sonst gewendelten Struktur der Funktionsleiter geradlinig laufend).
- Die Zuleitung vom Sensor zur Auswerteeinheit ist zwecks Verminderung der Kopplung an den Funktionsleiter oder der externen Störungen als elektrischer Leiter ausgebildet, wobei die Verminderung der Kopplung durch geeignete Materialien realisiert wird, z. B. Hochwiderstandsdrähte, Ionenleiter mit hohen Widerstand (entsprechend wenig leitfähiger Elektrolyt) o. ä.
- In einer bevorzugten Ausführung sind der Sensor und die Zuleitung zur Auswerteeinheit aus dem gleichen Material, ohne Verbindungstechnik. (Realisierungsbeispiel: Aus ein und desselben Stück Widerstandsdraht, aus dem die Zuleitung besteht, ist auch die Sensorspule gewickelt.)
- Die Kodierung ist realisiert als FM PWM, Phasen- oder Polarisationskodierung.

Vorteile und Zweckmäßigkeiten der Erfindung ergeben sich im Übrigen aus der nachfolgenden skizzenartigen Beschreibung von Ausführungsbeispielen anhand der Figuren. Von diesen zeigen:
- Fig. 1A und 1B: zwei Varianten einer Ausführungsform der Erfindung als schematische Querschnittsdarstellungen,
- Fig. 1C: eine Prinzipskizze einer der Elektrodenkathetereinrichtung nach Fig. 1A oder 1B zuzuordnenden Auswertungseinheit,
- Fig. 1D: eine Detailansicht eines in den Elektrodenkathetereinrichtung gemäß Fig. 1A oder 1B eingesetzten Stromsensors und
- Fig. 1E: eine Prinzipskizze einer Ausführung des in dem Stromsensor eingesetzten Messelementes sowie
- Fig. 1F: eine Prinzipskizze eines alternativ einsetzbaren Messelementes,
- Fig. 2: eine Prinzipskizze eines in einer erfindungsgemäßen Elektrodenkathetereinrichtung einsetzbaren elektrisch-optischen Modulators und
- Fig. 3: ein Funktions-Blockschaltbild einer erfindungsgemäßen elektromedizinischen Anordnung.

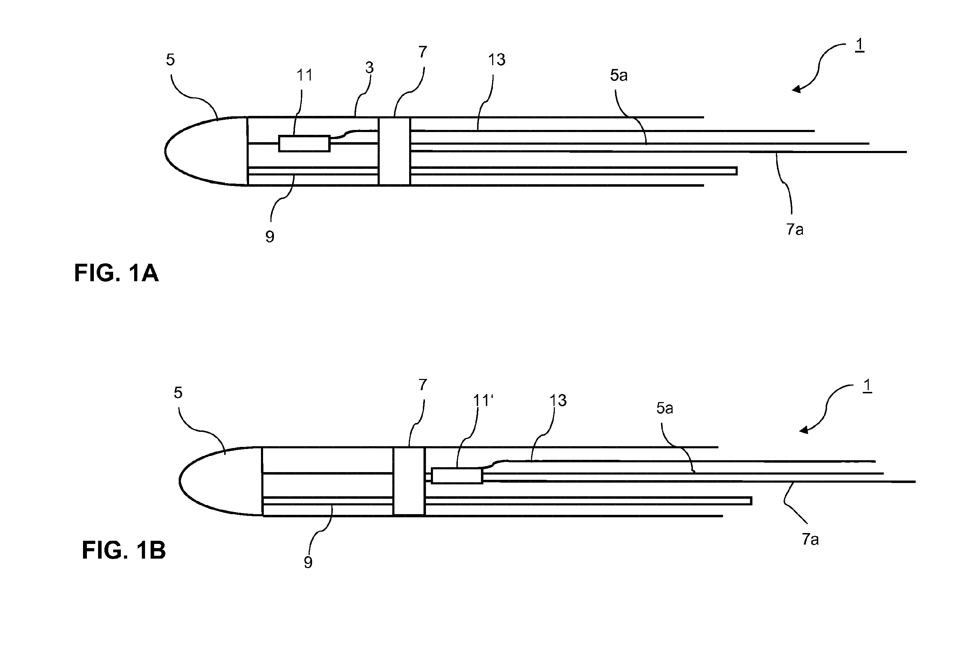

Fig. 1A zeigt schematisch das distale Ende einer Schrittmacherelektrodenleitung 1, die in einem Leitungskörper 3 eine Spitzenelektrode 5 und eine hiervon beabstandete Ringelektrode 7 mit zugehörigen Zuleitungen 5a und 7a sowie einen Spülkanal 9 umfasst. Zur Strommessung in der Zuleitung 5a zur Spitzenelektrode 5 ist benachbart zu dieser ein Stromsensor 11 vorgesehen, an den als Übertragungskanal ein Lichtwellenleiter 13 angeschlossen ist. Eine modifizierte Elektrodenleitung 1' gemäß Fig. 1B ist im Prinzip gleichartig aufgebaut, enthält aber einen zur Strommessung in beiden Zuleitungen 5a, 7a ausgebildeten und entsprechend anders platzierten Stromsensor 11'.

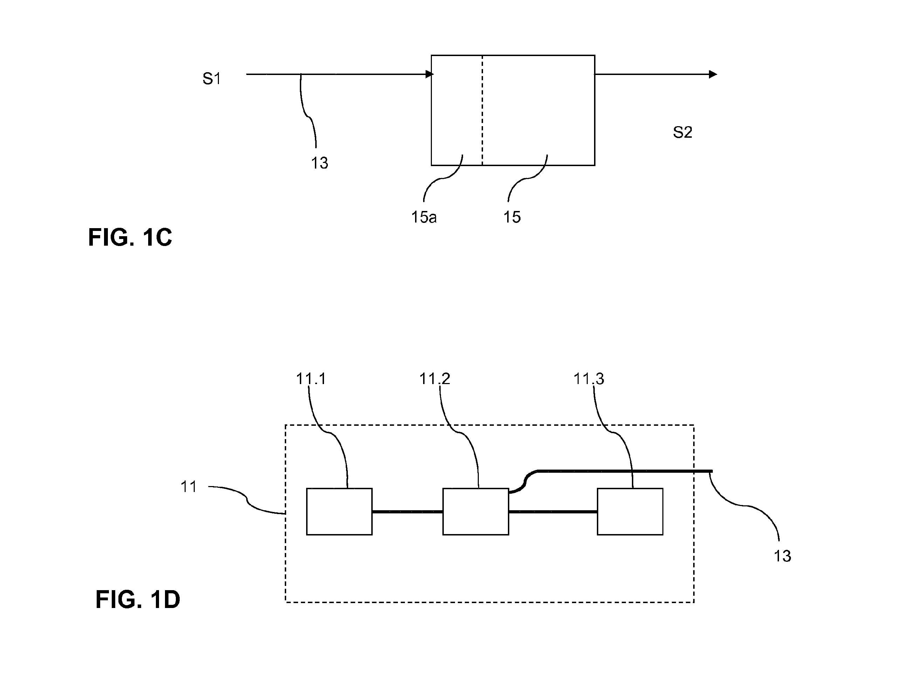

Fig. 1C zeigt gewissermaßen das Ende des Signalübertragungskanals, nämlich eine mit dem Lichtwellenleiter 13 verbundene Auswertungseinheit 15, an deren Eingang ein optisch/elektrischer Wandler 15a zur Rückwandlung des auf optischem Wege übertragenen Messsignals vorgesehen ist und die aus einem Messsignal S 1 ein Auswertungsergebnis S2 erzeugt, welches zur Steuerung eines Mess- bzw. Therapievorganges genutzt werden kann.

Die Auswertungseinheit 15 wandelt das optische wieder in ein elektrisches Signal und Filtert das Signal nach Frequenzen. So kann ein Strom im kHz-Bereich, der therapeutischen Nutzen hat (z.B. bei der Ablation), von einem vom MRT induzierten HF-Strom im MHz-Bereich gut unterschieden werden. Bei der Detektion eines HF-induzierten Stromes im MHz-Bereich kann die Auswerteeinheit entsprechende Warnsignale senden und so eine unerwünschte Gewebeerhitzung verhindern. Diese Signale können direkt für Gegenmaßnahmen verwendet werden (Closed Loop). So kann z.B. die HF-Leistung für die Bildgebung abgeschaltet oder reduziert werden. Alternativ kann die resultierende Erwärmung mit der Kenntnis des Stromes über die Spülung des Katheters gekühlt werden. Mit der Kenntnis vom Zusammenhang zwischen dem austretenden HF-Strom und der Gewebeerwärmung kann die Menge und der Durchfluss der Spülung zur Kühlung Situationsbedingt automatisch geregelt werden. Weiterhin erkennt die Auswerteeinheit den Ausfall des optischen Stromsensors unmittelbar, so dass ein sicheres Beobachten des induzierten HF-Stroms sichergestellt wird. Dies ist möglich, da der optische Modulator die Laserleistung im CW-Modus betreibt. Ein Ausfall der Laserdiode kann so unmittelbar durch Ausfall der optischen Leistung im Lichtwellenleiter vom Empfänger an der Auswerteeinheit detektiert werden.

Ein möglicher Aufbau des Stromsensors 11 bzw. 11' aus Fig. 1A bzw. 1B ist in Fig. 1D skizziert. Der Stromsensor besteht demnach aus drei Funktionsblöcken, nämlich einem Messelement 11.1 (im engeren Sinne), einem elektrisch/optischen Modulator 11.2 und einem Energieversorgungsblock 11.3 für den Modulator 11.2.

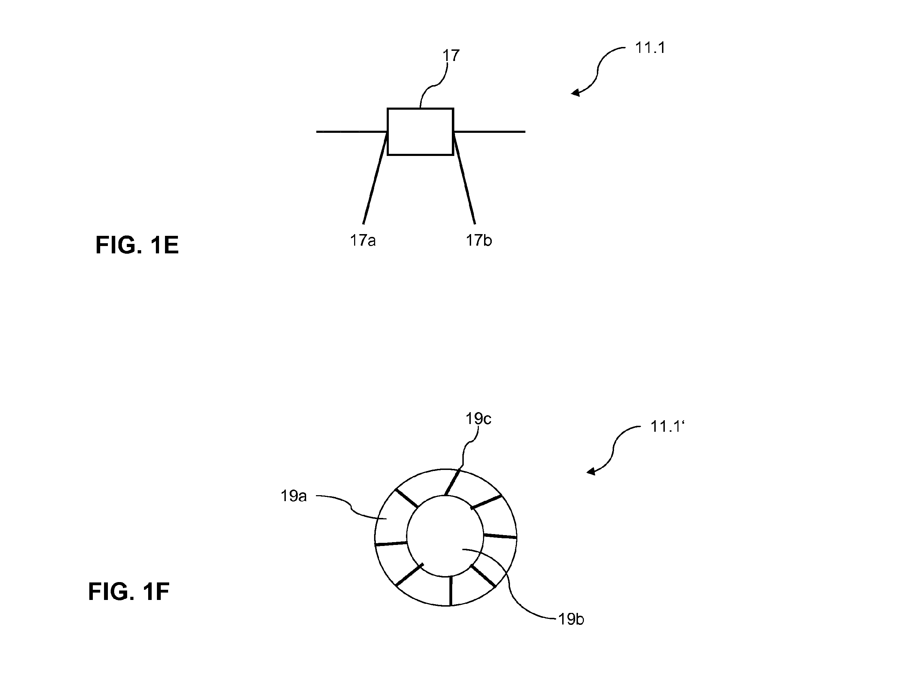

Fig. 1E zeigt skizzenhaft eine Ausführung des Messelementes 11.1 als ein in die Zuleitung 5a gemäß Fig. 1A eingefügten Widerstand 17 mit zugeordneten Leitungen 17a, 17b zum Abgreifen einer über dem Widerstand abfallenden Spannung, die als Messsignal genutzt werden kann. Fig. 1F zeigt, als alternative Ausführung 11.1' des Stromsensors eine Toroidspule, mit einem Spulenkörper 19a, der eine Leitungsdurchführung 19b zum Durchtritt der Leitung, auf der der Stromfluss gemessen werden soll, und eine Spulenwicklung 19c umfasst. Für den praktischen Einsatz sollte die Spule gegen die B1-Felder des MRT abgeschirmt werden, damit es nicht zu einer unerwünschten Fehldetektion kommt. Die Schirmung sollte dabei entlang des inneren Ringes geschlitzt sein, wodurch man eine so genannte Rogowskispule erhält. So ist die Spule sensitiv auf durchgeführte stromführende Leiter und schirmt von außen wirkenden Feldern effektiv ab.

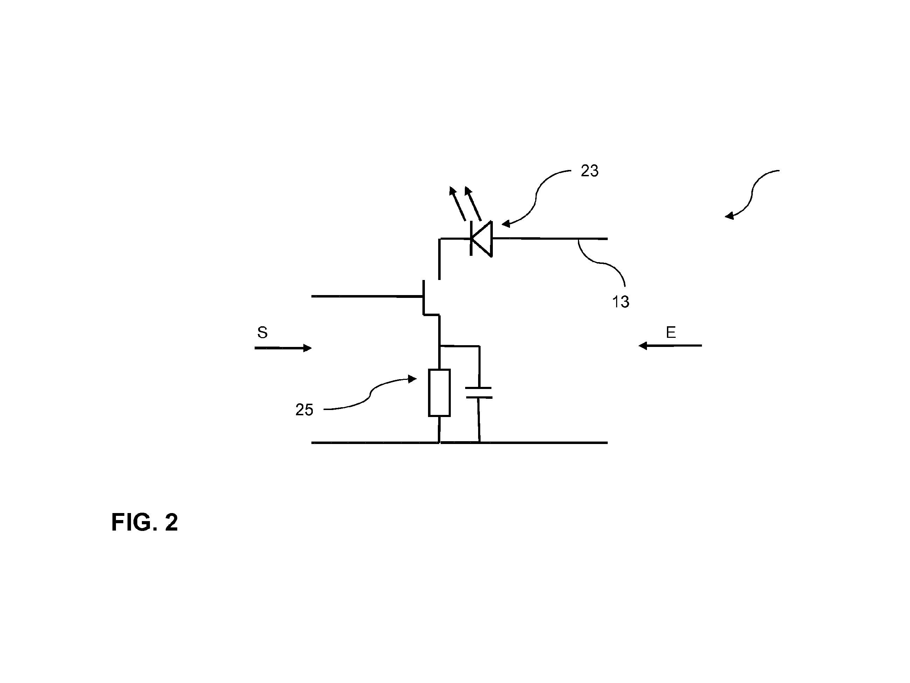

Fig. 2 zeigt ― wiederum nur skizzenartig ― eine Realisierung des elektrisch/optischen Modulators 11.2 aus Fig. 1D mit einem Transistor 21, einer Laserdiode 23 und einem RC-Glied 25. Mit dem Pfeil S ist der Signaleingang bezeichnet und mit dem Pfeil E die Energieversorgungs-Seite der Modulatoranordnung. Die Energieversorgung ist z. B. als optische Energieversorgung über ein photovoltaisches Element oder als elektrische Stromversorgung über einen Hochwiderstandsdraht oder auch auf andere Weise möglich. Mit Hochwiderstandsdrähten ist nach ersten Erkenntnissen der Anmelderin eine störungsarme Energieversorgung möglich, und der Aufwand zur Integration in die Elektrodenkathetereinrichtung ist nach derzeitigem Erkenntnisstand gegenüber einer optischen Energieversorgung niedriger.

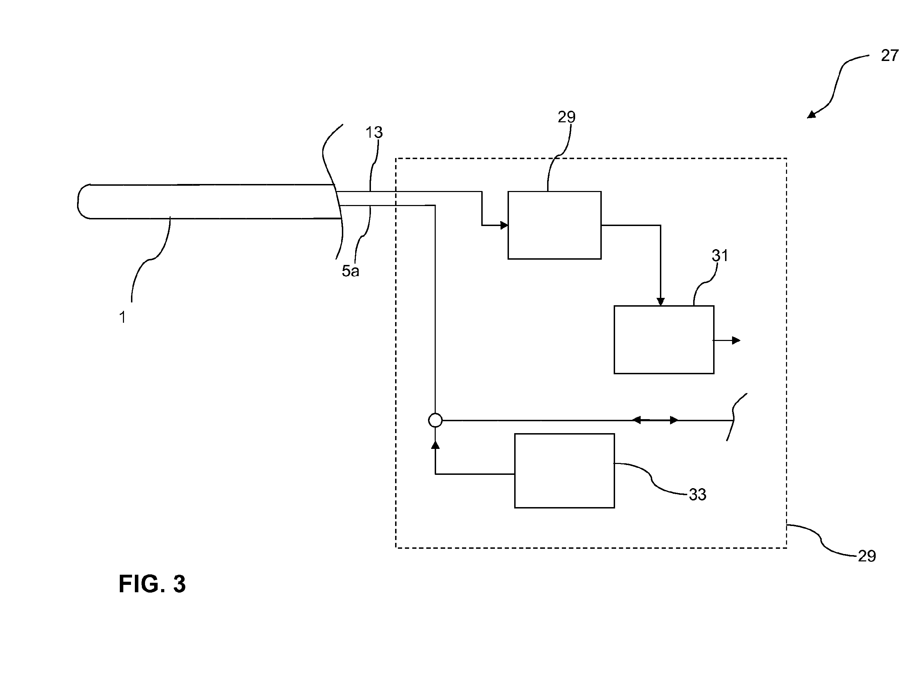

Fig. 3 zeigt ― wiederum schematisch in Art eines Funktions-Blockschaltbildes ― den Grundaufbau einer erfindungsgemäßen elektromedizinischen Vorrichtung 27, hier speziell einer Schrittmacher-Anordnung, die eine Schrittmacherleitung 1 (Fig. 1A) und einen erfindungsgemäß angepassten Herzschrittmacher 29 umfasst. In diesem sind neben den Standard-Komponenten eines Herzschrittmachers (hier nicht dargestellt) ein an den Lichtwellenleiter 13 angeschlossener Auswertungsblock 15 (siehe auf Fig. 1C) ein an den Auswertungsblock 15 ausgangsseitig angeschlossener Steuerblock 31 zur Beeinflussung der Schrittmachertherapie in Abhängigkeit von den Signalen des in die Schrittmacherleitung integrierten Stromsensors und schließlich ein Sensor-Stromversorgungsblock 33 integriert, der in diesem Beispiel ausgangsseitig an die Elektrodenzuleitung 5a angeschlossen ist. Diese stellt also zugleich den Energieübertragungskanal der Anordnung dar, was eine geeignete (aber im Rahmen fachmännischen Wissens zu lösende) Entkopplung gegenüber den auf der gleichen Leitung übertragenen Therapiesignalen erfordert.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Elektrodenkathetereinrichtung, die eine Leiterstruktur mit mindestens einer Elektrode enthält, mit Strommessmitteln zur Messung eines durch ein äußeres elektromagnetisches Feld in der Leiterstruktur induzierten Stromes, wobei die Strommessmittel zur lokalen Strommessung, insbesondere von Stromstärken und -phasen in der oder einer Elektrode oder einem vorbestimmten Bereich der Leiterstruktur ausgebildet und an einen mit dem äußeren Feld nicht wechselwirkenden Signalübertragungskanal angeschlossen sind.

2. Elektrodenkathetereinrichtung nach Anspruch 1, wobei den Strommessmitteln ein elektrisch/mechanischer Wandler zur Signalwandlung in ein mechanisches Signal zugeordnet und der Signalübertragungskanal zur mechanischen Signalübertragung ausgebildet ist.

3. Elektrodenkathetereinrichtung nach Anspruch 1, wobei den Strommessmitteln ein elektrisch/optischer Wandler zur Signalwandlung in ein optisches Signal zugeordnet und der Signalübertragungskanal zur optischen Signalübertragung ausgebildet ist.

4. Elektrodenkathetereinrichtung nach Anspruch 1, wobei den Strommessmitteln ein elektrisch/thermischer Wandler zur Signalwandlung in ein thermisches Signal zugeordnet und der Signalübertragungskanal zur thermischen Signalübertragung ausgebildet ist.

5. Elektrodenkathetereinrichtung nach Anspruch 1, wobei den Strommessmitteln ein elektrisch/elektrischer Wandler bzw. Modulator zur Wandlung des Ausgangssignals der Strommessmittel in bzw. zur Aufmodulation auf elektromagnetische Wellen in einem Frequenzbereich zugeordnet ist, der mit einem Frequenzspektrum des äußeren Feldes nicht wesentlich interferiert, und der Signalübertragungskanal zur elektromagnetischen Signalübertragung ausgebildet ist.

6. Elektrodenkathetereinrichtung nach Anspruch 5, wobei der Signalübertragungskanal einen drahtlosen Übertragungsabschnitt aufweist.

7. Elektrodenkathetereinrichtung nach Anspruch 5 oder 6, wobei dem elektrisch/elektrischen Wandler bzw. Modulator Kodierungsmittel zur Kodierung des Ausgangssignals der Strommessmittel zur Verringerung von Störeinflüssen aus dem äußeren elektromagnetischen Feld zugeordnet sind.

8. Elektrodenkathetereinrichtung nach einem der Ansprüche 5 bis 7, wobei der Signalübertragungskanal einen elektrischen Leiter aufweist, welcher in Abstimmung auf die geometrische Ausführung der Leiterstruktur aus derartigem Material gebildet und/oder derart geformt und/oder derart verlegt ist, dass nur eine geringe Kopplung mit dem äußeren elektromagnetischen Feld und der Leiterstruktur erfolgt.

9. Elektrodenkathetereinrichtung nach Anspruch 1, wobei den Strommessmitteln unmittelbar ein elektrischer Leiter als elektrischer Signalübertragungskanal oder Teil eines solchen zugeordnet ist, wobei die Strommessmittel und der zugeordnete Leiter insbesondere einstückig ausgebildet sind.

10. Elektrodenkathetereinrichtung nach einem der vorangehenden Ansprüche, wobei die Strommessmittel als passive oder energie-autark arbeitende Messmittel ausgebildet sind.

11. Elektrodenkathetereinrichtung nach einem der Ansprüche 1 bis 9, wobei die Strommessmittel als aktive Messmittel ausgebildet und an einen Energieübertragungskanal angeschlossen sind.

12. Elektrodenkathetereinrichtung nach Anspruch 11, wobei der Energieübertragungskanal einen drahtlosen Übertragungsabschnitt aufweist.

13. Elektrodenkathetereinrichtung nach Anspruch 11 oder 12, wobei der Energieübertragungskanal einen elektrischen Leiter, insbesondere Elektroden- oder Ionenleiter, aufweist.

14. Elektrodenkathetereinrichtung nach Anspruch 11, wobei der Energieübertragungskanal Mittel zur mechanischen, insbesondere hydraulischen oder pneumatischen, Energieübertragung aufweist.

15. Elektrodenkathetereinrichtung nach Anspruch 11, wobei der Energieübertragungskanal Mittel zur optischen Energieübertragung, insbesondere eine Lichtleitfaser, aufweist.
